# EUROPEAN PATENT SPECIFICATION

(11) **EP 2 995 956 B1**
(45) Date of publication and mention of the grant of the patent: **15.11.2017**
(21) Application number: 14794106.6
(22) Date of filing: 08.05.2014
(51) Int. Cl.: G01N 33/68, C12Q 1/68, G01N 33/92

(54) **USE OF A DIAGNOSTIC MARKER COMPOSITION COMPRISING APOLIPOPROTEIN M FOR DIAGNOSING ALZHEIMER'S DISEASE**
VERWENDUNG EINER DIAGNOSEMARKERZUSAMMENSETZUNG MIT APOLIPOPROTEIN M ZUR DIAGNOSE VON MORBUS ALZHEIMER
UTILISATION D'UNE COMPOSITION DE MARQUEUR DE DIAGNOSTIC COMPRENANT DE L'APOLIPOPROTÉINE M POUR LE DIAGNOSTIC DE LA MALADIE D'ALZHEIMER

(30) Priority: 08.05.2013 KR 20130052019
(43) Date of publication of application: 16.03.2016
(73) Proprietor: Kyungpook National University Industry-Academic Cooperation Foundation, Daegu 702-701 (KR)
(72) Inventor: BAE, Jae Sung, Daegu 701-796 (KR); JIN, Hee Kyung, Daegu 705-020 (KR)
(74) Representative: Docherty, Robert Charles
(86) International application number: PCT/KR2014/004058
(87) International publication number: WO 2014/182072

(56) References cited:
- EP-A1- 1 434 053
- US-A1- 2003 113 808
- US-A1- 2013 023 428
- US-B2- 8 389 222
- HU Y W ET AL: "Characteristics of apolipoprotein M and its relation to atherosclerosis and diabetes", BIOCHIMICA AND BIOPHYSICA ACTA. MOLECULAR AND CELL BIOLOGY OF LIPIDS, ELSEVIER, AMSTERDAM, NL, vol. 1801, no. 2, 1 February 2010 (2010-02-01), pages 100-105, XP026826592, ISSN: 1388-1981 [retrieved on 2009-11-10]
- AMRO KABBARA ET AL: "Exclusion of CYP46 and APOM as candidate genes for Alzheimer's disease in a French population", NEUROSCIENCE LETTERS, vol. 363, no. 2, 1 June 2004 (2004-06-01), pages 139-143, XP055297245, IE ISSN: 0304-3940, DOI: 10.1016/j.neulet.2004.03.066
- ORDONEZ ET AL.: 'Gender differences in apolipoprotein D expression during aging and in Alzheimer disease' NEUROBIOLOGY OF AGING vol. 33, no. 2, 2012, pages 433.E11 - 433.E20, XP055291490
- VERGHESE ET AL.: 'Apohpoprotein E in disease and other nemological disorders' THE LANCET NEUROLOGY. vol. 10, no. 3, 2011, pages 241 - 252, XP055291491

## Description

### Technical Field

It is an object of the present invention to provide the use of the biomarker apolipoprotein M for diagnosing Alzheimer's disease, the use of a composition for diagnosing Alzheimer's disease, said composition comprising a formulation for measuring a protein expression level of apolipoprotein M or an mRNA expression level of a gene coding apolipoprotein M, the use of a kit comprising said composition for diagnosing Alzheimer's disease , and a method for providing information for diagnosing Alzheimer's disease using the composition.

### Background Art

Alzheimer's disease is known as caused by death of neurons by extracellular β-amyloid deposits, which are senile plaques, and intercellular hyperphosphorylated tau proteins throughout the brain. Alzheimer's disease causes gradual memory impairment, cognitive deficit, changes in individual personality, etc. Once Alzheimer's disease occurs, the disease continuously proceeds. Also, methods for diagnosing Alzheimer's disease are restrictive, and there is no fundamental therapy for the disease. Furthermore, it is known that a group with the disease is known as having reduced average life expectancy per age group compared to a normal group.

Alzheimer's disease causes dementia which is the most common disease among the elderly. In Korea, as the age increases by 5 in the age range of 65 to 85, the incidence rate of Alzheimer's disease increases twice each. Additionally, the number of the elderly with dementia is estimated to be about 0.47 million in 2010, which accounts for 8.8% of the elderly population over 65. Considering the rapid aging rate in Korea, the number of patients with dementia will also rapidly increase. In future, the number of patients with dementia is estimated to be 0.75 million in 2020, 1.14 million in 2030, and 2.13 million in 2050.

Currently, cholinesterase inhibitors (Aricept, Exelon, Reminyl, etc.) targeting for cholinergic neuron, and Memantine, which is a glutamate antagonist, are drugs for preventing or treating Alzheimer's disease. These drugs were made to slow down the progress of Alzheimer's disease or to treat symptoms of Alzheimer's disease, and are prescribed after an exact confirmation thereon is made. Most Alzheimer's disease could be diagnosed when the disease proceeds to some degree and symptoms thereof are shown. Accordingly, an early diagnosis from which a disease could be confirmed before a symptom thereof is shown has been difficult to be made as to Alzheimer's disease. Hu et al (Biochimica et Biopysica Acta volume 1801, (2010), pp100-105) characterizes apolipoprotein M and its association with atherosclerosis and diabetes but is silent to its association with Alzheimer's disease. EP1434053A1 discloses the association of apolipoprotein genes E2, E3 and E4 and Alzheimer's disease. Kabbara et al (Neuroscience Letters volume 363, (2004), pp139-143) discloses biomarkers that are not associated with Alzheimer's disease of which one excluded candidate gene is apolipoprotein M.

Thus, new means for early diagnosis related with early diagnosis methods for Alzheimer's disease are keenly required.

Accordingly, the present inventors have continuously conducted a research on the development of a marker for diagnosing Alzheimer's disease, confirmed that patients with Alzheimer's diseases have a significantly reduced expression of apolipoprotein M compared to normal people, and completed the present invention.

### Summary of invention

It is an object of the present invention to provide the use of the biomarker apolipoprotein M for diagnosing Alzheimer's disease.

It is another object of the present invention to provide the use of a composition for diagnosing Alzheimer's disease comprising a formulation for measuring a protein expression level of apolipoprotein M or an mRNA expression level of a gene coding apolipoprotein M.

It is another object of the present invention to provide the use of a kit comprising said composition for diagnosing Alzheimer's disease.

It is another object of the present invention to provide a method for providing information for diagnosing Alzheimer's disease using the composition.

In order to achieve the above-mentioned objects, the present invention provides the use of the biomarker apolipoprotein M for diagnosing Alzheimer's disease.

Also, the present invention provides the use of a composition comprising a formulation for measuring a protein expression level of apolipoprotein M or an mRNA expression level of a gene coding apolipoprotein M for diagnosing Alzheimer's disease. The formulation for measuring the protein expression level is an antibody specific for apolipoprotein M. The formulation for measuring the mRNA expression level is at least one selected from the group consisting of an antisense oligonucleotide, a primer, and a probe which specifically bind to a gene of apolipoprotein M.

Also, the present invention provides the use of a kit comprising a composition for diagnosing Alzheimer's disease.

Also, the present invention provides a method for providing information for diagnosing Alzheimer's disease using the composition.

Since an expression of apolipoprotein M according to the present invention is significantly reduced in patients with Alzheimer's disease compared to normal people, apolipoprotein M may be useful for accurate and fast diagnosis on the incidence of Alzheimer's disease.

### Brief description of drawings

Fig. 1 is a view illustrating a numerical value of apolipoprotein M in plasma obtained from patients with Alzheimer's disease and from a normal control group;
Fig. 2 is a view illustrating a numerical value of apolipoprotein M in HDL fractionated from plasma of patients with Alzheimer's disease and of a normal control group;
Fig. 3 is a view illustrating a numerical value of apolipoprotein M in plasma of model mice with Alzheimer's disease and of a normal control group mice; and
Fig. 4 is a view illustrating a numerical value of apolipoprotein M in HDL fractionated from plasma of model mice with Alzheimer's disease and of a normal control group mice.

### Best mode for carrying out the invention

Hereinafter, the present invention will be explained in detail.

The present invention provides the use of the biomarker apolipoprotein M for diagnosing Alzheimer's disease. Also, the present invention provides the use of a composition comprising a formulation for measuring a protein expression level of apolipoprotein M or an mRNA expression level of a gene coding apolipoprotein M for diagnosing Alzheimer's disease, wherein, the formulation for measuring the protein expression level is an antibody specific for apolipoprotein M, or the formulation for measuring the mRNA expression level is at least one selected from the group consisting of an antisense oligonucleotide, a primer, and a probe which specifically bind to a gene of apolipoprotein M.

The term "diagnosis" used herein means confirmation of the presence or characteristics of pathological conditions. With regard to the purposes of the present invention, the diagnosis means confirmation on whether Alzheimer's disease occurs or whether Alzheimer's disease is likely to occur.

The term "marker" used herein means a material capable of diagnosing Alzheimer's disease, and includes organic biomolecules, such as polypeptide, nucleic acids (e.g., mRNA, etc.), lipid, glycolipid, glycoprotein, sugar (monosaccharides, disaccharides, oligosaccharides, etc.) related with Alzheimer's disease. With regard to the purposes of the present invention, the marker is apolipoprotein M which is a protein whose expression is reduced in patients with Alzheimer's disease.

The term "measurement on a protein expression level" used herein is a process of confirming the presence or expression level of apolipoprotein M, which is a protein related with Alzheimer's disease in a biological sample for diagnosing Alzheimer's disease, and is made by measuring an amount of protein. The analysis method includes western blot, enzyme linked immunosorbent assay (ELISA), radioimmunoassay (RIA), radioimmunodiffusion, Ouchterlony immunodiffusion, rocket immunoelectrophoresis, immunohistostaining, immunoprecipitation assay, complement fixation assay, fluorescence activated cell sorter (FACS), protein chip, etc., but is not limited thereto. A formulation for measuring a protein expression level of the present invention is an antibody.

The term "antibody" used herein means a specific protein molecule instructed for an antigenic site. With regard to the purposes of the present invention, the antibody means an antibody which is specifically binds to apolipoprotein M, and includes all of a polyclonal antibody, a monoclonal antibody, and a recombinant antibody.

The term "measurement on an mRNA expression level" used herein means measurement on the presence and expression level of mRNA of a gene coding apolipoprotein M, which is a protein related with Alzheimer's disease, in a biological sample for diagnosing Alzheimer's disease. The analysis method includes, for example, reverse transcription polymerase chain reaction (RT-PCR), competitive RT-PCR, realtime RT-PCR, RNase protection assay (RPA), Northern blotting, DNA chip, etc., but is not limited thereto. A formulation for measuring an mRNA level of a gene according to the present invention is an antisense oligonucleotide, a primer, or a probe.

The term "antisense" used herein refers to an oligomer with a sequence of nucleotide bases and a backbone between sub-units, where an antisense oligomer is hybridized with a target sequence in RNA by the formation of Watson-Crick base pair, typically allowing the formation of mRNA and RNA: hetero-oligomer duplex. The oligomer may have an exact sequence complementarity to the target sequence or near complementarity. The antisense oligomer may block or inhibit the translation of mRNA, and change the processing procedure of mRNA to produce a splice variant of the mRNA.

The term "primer" means a short nucleic acid sequence with short free 3' terminal hydroxyl group capable of forming a base pair with a complementary template and functioning as a starting point for template strand duplication. A primer may initiate DNA synthesis in the presence of a reagent for polymerization (i.e., DNA polymerase or reverse transcriptase) in a proper buffer solution at a proper temperature and 4 different nucleoside triphosphates.

The term "probe" means a fragment of nucleic acid such as RNA or DNA, etc., which is ones to hundreds of bases pairs capable of specifically binding to mRNA. Since a probe is labeled, the presence of specific mRNA may be confirmed. A probe may be prepared in the form of an oligo nucleotide probe, a single-stranded DNA probe, a double stranded DNA probe, an RNA probe, etc. Selection of proper probes and hybridization conditions may be modified based on those disclosed in the related art.

Since nucleic acid sequences of genes expressing apolipoprotein M, which is a protein related with Alzheimer's disease, are registered at a gene bank, a person skilled in the art may design an antisense oligonucleotide, a primer, or a probe which specifically amplifies a specific region of the genes based on the sequences.

The antisense oligonucleotide, primer, or probe of the present invention may be chemically synthesized using a phosphoramidite solid substrate method, or other methods widely known in the art. These nucleic acid sequences may be modified using various means disclosed in the related field. As non-restrictive examples of the modification, there are methylation, capsulation, substitution into at least one homologue of natural nucleotide, and modification between nucleotides, for example, modification to uncharged linkages (e.g., methylphosphonate, phosphotriester, phosphoroamidate,carbamate, etc.) or charged linkages (e.g., phosphorothioate, phosphorodithioate, etc.).

Since the expression of apolipoprotein M according to the present invention is significantly reduced in patients with Alzheimer's disease compared to normal people, apolipoprotein M may be useful for exact and rapid diagnosis on the incidence of Alzheimer's disease. Also, the present invention provides the use of a kit comprising said composition for diagnosing Alzheimer's disease.

The kit may be used to diagnose Alzheimer's disease by confirming a protein expression level of apolipoprotein M or an mRNA expression level of a gene coding apolipoprotein M. The kit may further include one or more types of other component compositions, solutions or devices, as well as primers, probes, antibodies, etc. for diagnosing Alzheimer's disease. The kit may be preferably in the form of a RT-PCR kit, a microarray chip kit, a DNA chip kit, and a protein chip kit, but is not limited thereto.

As a specific example, the kit for measuring the mRNA expression level of the gene coding apolipoprotein M may be a kit comprising essential elements necessary for performing RT-PCR. The RT-PCR kit may include test tubes or other suitable containers, reaction buffer solutions, deoxynucleotide (dNTPs), enzyme such as Taq-polymerase and reverse transcriptase, DNase and RNase inhibitors, DEPC-water, sterilized water, etc., other than each primer which is specific for marker genes.

The kit may also be a kit including essential elements necessary for performing a microarray. The microarray kit may include a substrate where a gene or cDNA, which is a fragment of the gene, is attached as a probe, and the substrate may include a gene of a quantitative control group or cDNA, which is a fragment of the gene. The microarray kit may be prepared using a marker of the present invention according to a manufacturing method commonly used in the related field. In order to prepare the microarray, it is preferable to use a micropipetting method using a piezoelectric manner in order to fix a DNA chip on the substrate using the searched marker as DNA molecule, or a method using a spotter in the shape of pin, etc., but is not limited thereto. The substrate of the microarray chip is preferably a chip coated with an active group selected from the group consisting of amino-silane, poly-L-lysine and aldehyde, but is not limited thereto. Also, the substrate is preferably selected from the group consisting of slide glass, plastic, metal, silicone, nylon membrane, and nitrocellulose membrane, but is not limited thereto.

The kit may also include essential elements necessary for performing a DNA chip. The DNA chip kit may include a substrate where a gene or cDNA, which is a fragment of the gene, or oligonucleotide is attached, a reagent, a formulation, an enzyme, etc. for preparing fluorescent probes. Also, the substrate may include a control group gene or cDNA, which is a fragment of the gene, or oligonucleotide.

Also, the present invention provides a method for providing information for diagnosing Alzheimer's disease, which includes the following steps:
(a) measuring a protein expression level of apolipoprotein M or an mRNA expression level of a gene coding apolipoprotein M from a biological sample;
(b) comparing the protein expression level of apolipoprotein M or the mRNA expression level of the gene coding apolipoprotein M of the biological sample with the protein expression level of apolipoprotein M or the mRNA expression level of the gene coding apolipoprotein M of a normal sample; and
(c) predicting or diagnosing that Alzheimer's disease occurs, when the protein expression level of apolipoprotein M or the mRNA expression level of the gene coding apolipoprotein M of the biological sample is more reduced than the protein expression level of apolipoprotein M or the mRNA expression level of the gene coding apolipoprotein M of the normal sample.

The term "biological sample" used herein includes whole blood, serum, plasma, saliva, urine, sputum, lymph, cell, etc., which are separated from an individual, but is not limited thereto.

A method for measuring a protein expression level according to the present invention includes western blot, enzyme linked immunosorbent assay (ELISA), radioimmunoassay (RIA), radioimmunodiffusion, Ouchterlony immunodiffusion, rocket immunoelectrophoresis, immunohistostaining, immunoprecipitation assay, complement fixation assay, fluorescence activated cell sorter (FACS), protein chip, etc., but is not limited thereto.

A method for measuring an mRNA expression level according to the present invention includes reverse transcription polymerase chain reaction (RT-PCR), competitive RT-PCR, realtime RT-PCR, RNase protection assay (RPA), Northern blotting, DNA chip, etc., but is not limited thereto.

Since the expression of apolipoprotein M is significantly reduced in patients with Alzheimer's disease compared to normal people, diagnosis on whether Alzheimer's disease occurs may be made exactly and rapidly.

Hereinafter, the present invention will be explained in detail with reference to embodiments. The embodiments are simply to explain the present invention more specifically. Thus, according to the gist of invention, it would be obvious to a skilled person in the art in the related field that the scope of invention is not limited by the embodiments.

### Example 1. Experimental materials and experimental methods

### 1-1. Blood collection and plasma separation from patients with Alzheimer's disease

Blood was collected from 24 patients diagnosed with Alzheimer's disease and a normal control 10 people. Each blood sample was centrifuged at 3,000 rpm for 15 minutes to divide the blood sample into blood corpuscle and plasma, and the plasma of a supernatant was separated. A plasma sample before being used for analysis was kept at -80°C.

### 1-2. Blood collection and plasma separation of model mice with Alzheimer's disease

As model mice with Alzheimer's disease, transgenic mice overexpressing hAPP695swe (APPswe) and presenilin-1M146V (PS1) mutants were used. They are mouse lines produced by GlaxoSmithKline (Harlow, UK) with a standard technology for C57BL/6 background (Charles River, UK), and APPswe mice were backcrossed with purebred C57BL/6 background mice, and crossed with PS1 mice, to produce double heterozygote mutant mice (APP/PS1). The mice used in the experiment were 3-, 6-, 9-, 12- and 15-month-old mice. Blood was collected from model mice with Alzheimer's disease (AD) and normal control mice (WT). More specifically, mice were anesthetized, and 500 µl to 700 µl of blood was collected from the heart and was put in a heparin tube (BD Falcon). Then, each blood sample was centrifuged at 1,200 rpm for 5 minutes to separate plasma of the supernatant. A plasma sample before being used for analysis was kept at -80°C.

### 1-3. HDL fraction in plasma

60 µl of plasma sample was put in a tube for ultracentrifugation, the same amount of PBS (Gibco) solution was added thereto to form a layer onto the plasma, and was centrifuged at 4°C, 70,000 rpm for 3 hours by using an ultracentrifuge (HITACHI cp100wx Centrifuge P70AT rotor). In the sample separated into two layers, 60 µl of the bottom layer was transferred to a new tube for ultracentrifugation, the same amount of NaBr (Sigma-Aldrich) solution (density = 1.12 g/ml) was added thereto, was mixed about five times using a pipette, and was centrifuged at 4 °C, 70,000 rpm for 18 hours by using the ultracentrifuge again. In the sample separated into two layers, HDL 60 µl of the bottom layer was transferred to a new tube, and the sample before being used for analysis was kept at -80°C.

### 1-4. Apo M ELISA (Enzyme-linked immunosorbent assay)

The amount of apolipoprotein M included in the plasma and fractionated HDL of human beings or mice and was quantified using a commercial ELISA kit (CUSABIO Human ApoM ELISA Kit and Mouse ApoM ELISA Kit). As a standard curve, a refined apolipoprotein M standard was used.

### Example 2. Numerical value change of apolipoprotein M in plasma and HDL fraction in patients with Alzheimer's disease

In order to find out a correlation between apolipoprotein M in blood and Alzheimer's disease, an experiment was carried out for comparing a numerical value of apolipoprotein M in plasma and HDL fraction of patients with Alzheimer's disease obtained from Example 1 with a numerical value of a normal control group.

### 2-1. Confirmation on numerical value of apolipoprotein M in plasma of patients with Alzheimer's disease

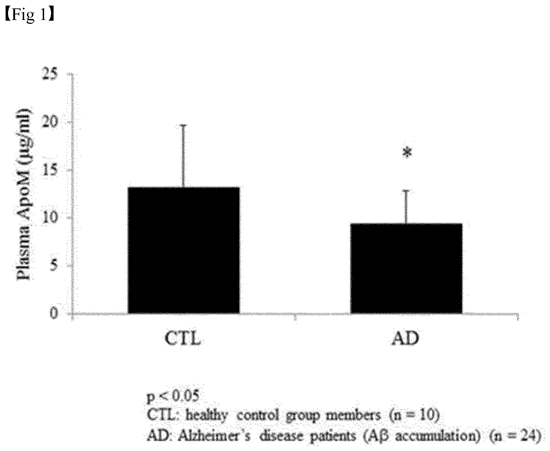

Blood was collected from patients with Alzheimer's disease and from people of the normal control group, the plasma was separated by the method mentioned in Example 1-1, and then the numeral value of apolipoprotein M in the plasma was quantitatively analyzed by the method mentioned in Example 1-4. A result thereof is shown in Fig. 1.

As shown in Fig. 1, it can be confirmed that the content of apolipoprotein M in the plasma of patients with Alzheimer's disease is significantly reduced compared to that in the plasma of people of the normal control group (p < 0.05, normal control group n = 10, patients with Alzheimer's disease n = 24).

### 2-2. Confirmation on numerical value of apolipoprotein M in HDL fraction of patients with Alzheimer's disease

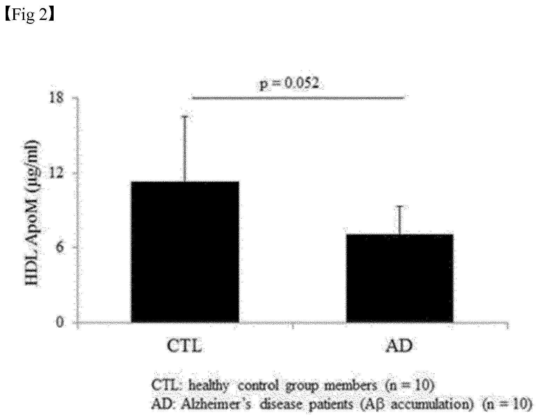

In the plasma separated from patients with Alzheimer's disease and people from a normal control group, HDL was fractionated and separated by the method mentioned in Example 1-3, and the numerical value of apolipoprotein M in the plasma was quantitatively analyzed by the method mentioned in Example 1-4. A result thereof is shown in Fig. 2.

As shown in Fig. 2, it can be confirmed that the numerical value of apolipoprotein M in HDL fraction of patients with Alzheimer's disease is significantly reduced compared to that in HDL of people of the normal control group (p = 0.052, n = 10).

### Example 3. Numerical change of apolipoprotein M in plasma and HDL fraction of model mice with Alzheimer's disease

In order to re-verify a correlation between apolipoprotein M in blood and Alzheimer's disease in model mice with Alzheimer's disease according to time, an experiment was carried out for comparing the numerical value of apolipoprotein M in the plasma and HDL fraction of model mice with Alzheimer's disease with that of mice of the normal control group.

### 3-1. Confirmation on numerical value of apolipoprotein M in plasma of model mice with Alzheimer's disease

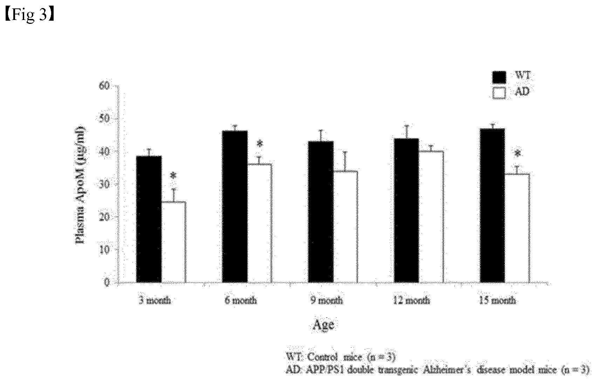

Blood was collected from 3-, 6-, 9-, 12- and 15-month-old model mice with Alzheimer's disease (AD) and mice from a normal control group (WT), plasma was separated by the method mentioned in Example 1-3, and then the numerical value of apolipoprotein M in plasma was quantitatively analyzed by the method mentioned in Example 1-4. A result thereof is shown in Fig. 3.

As shown in Fig. 3, it can be confirmed that the numerical value of apolipoprotein M in the plasma of model mice with Alzheimer's disease according to time is significantly reduced compared to that in the plasma of mice of the normal control group (* p < 0.05, n = 3).

### 3-2. Confirmation on numerical value of apolipoprotein M in HDL fraction of model mice with Alzheimer's disease

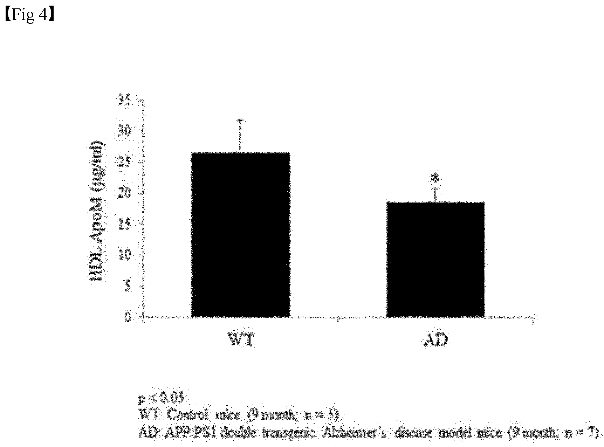

In the plasma of 9-month-old model mice with Alzheimer's disease and mice of the normal control group, HDL was fractionated and separated by the method mentioned in Example 1-3, and the numerical value of apolipoprotein M in the plasma was quantitatively analyzed by the method mentioned in Example 1-4. A result thereof is shown in Fig. 4.

As shown in Fig. 4, it can be confirmed that the content of apolipoprotein M in HDL fraction of model mice with Alzheimer's disease is significantly reduced compared to that in HDL of mice of the normal control group (p < 0.05, mice of the normal control group n = 5, model mice with Alzheimer's disease n = 7).

## Claims

1. Use in vitro of apolipoprotein M as biomarker for diagnosing Alzheimer's disease.

2. The use of claim 1, wherein an expression level of the apolipoprotein M in a sample with Alzheimer's disease is more reduced than an expression level of the apolipoprotein M in a normal sample.

3. Use in vitro of a composition comprising a formulation for measuring a protein expression level of apolipoprotein M or an mRNA expression level of a gene coding apolipoprotein M, for diagnosing Alzheimer's disease, wherein, the formulation for measuring the protein expression level is an antibody specific for apolipoprotein M, or the formulation for measuring the mRNA expression level is at least one selected from the group consisting of an antisense oligonucleotide, a primer, and a probe which specifically bind to a gene of apolipoprotein M.

4. Use of a kit comprising the composition of claim 3 for diagnosing Alzheimer's disease.

5. The use of claim 4, wherein the kit is at least one selected from the group consisting of a reverse transcription polymerase chain reaction (RT-PCR) kit, a microarray chip kit, a DNA kit, and a protein chip kit.

6. A method for providing information for diagnosing Alzheimer's disease, the method comprising:
(a) measuring a protein expression level of apolipoprotein M or an mRNA level of a gene coding apolipoprotein M from a biological sample;
(b) comparing the protein expression level of apolipoprotein M or the mRNA expression level of the gene coding apolipoprotein M of the biological sample with the protein expression level of apolipoprotein M or the mRNA expression level of the gene coding apolipoprotein M of a normal sample; and
(c) predicting or diagnosing that Alzheimer's disease occurs, when the protein expression level of apolipoprotein M or the mRNA expression level of the gene coding apolipoprotein M of the biological sample is more reduced than the protein expression level of apolipoprotein M or the mRNA expression level of the gene coding apolipoprotein M of the normal sample.

7. The method of claim 6, wherein the biological sample in the step (a) is at least one selected from the group consisting of whole blood, serum, plasma, saliva, urine, sputum, lymph, and cell, which are separated from an individual.

8. The method of claim 6, wherein the method for measuring the protein expression level in the step (a) is at least one selected from the group consisting of western blot, enzyme linked immunosorbent assay (ELISA), radioimmunoassay (RIA), radioimmunodiffusion, Ouchterlony immunodiffusion, rocket immunoelectrophoresis, immunohistostaining, immunoprecipitation assay, complement fixation assay, fluorescence activated cell sorter (FACS), and protein chip.

9. The method of claim 6, wherein the method for measuring the mRNA expression level in the step (a) is at least one selected from the group consisting of reverse transcription polymerase chain reaction (RT-PCR), competitive RT-PCR, realtime RT-PCR, RNase protection assay (RPA), Northern blotting, and DNA chip.

## Patentansprüche

1. In-Vitro-Verwendung von Apolipoprotein M als Biomarker zur Diagnose von Morbus Alzheimer.

2. Verwendung nach Anspruch 1, wobei ein Expressionsniveau von Apolipoprotein M in einer Probe mit Morbus Alzheimer verringerter als ein Expressionsniveau von Apolipoprotein M in einer normalen Probe ist.

3. In-Vitro-Verwendung einer Zusammensetzung, welche eine Formulierung zum Messen eines Proteinexpressionsniveaus von Apolipoprotein M oder eines mRNA-Expressionslevels eines Gens, welches für Apolipoprotein M codiert, umfasst, zur Diagnose von Morbus Alzheimer, wobei die Formulierung zum Messen des Proteinexpressionsniveaus ein Antikörper ist, welcher spezifisch für Apolipoprotein M ist, oder die Formulierung zum Messen des mRNA-Expressionsniveaus wenigstens eines, ausgewählt aus der Gruppe, bestehend aus einem Antisense-Oligonukleotid, einem Primer und einer Probe, welche spezifisch an ein Gen von Apolipoprotein M binden, ist.

4. Verwendung eines Kits, umfassend die Zusammensetzung nach Anspruch 3 zur Diagnose von Morbus Alzheimer.

5. Verwendung nach Anspruch 4, wobei das Kit mindestens eines, ausgewählt aus der Gruppe, bestehend aus einem Reverse Transkriptions-Polymerase-Kettenreaktion-(RT-PCR)-Kit, einem Microarray-Chip-Kit, einem DNA-Kit und einem Protein-Chip-Kit, ist.

6. Verfahren zum Bereitstellen von Informationen zur Diagnose von Morbus Alzheimer, wobei das Verfahren Folgendes umfasst:
(a) Messen eines Proteinexpressionsniveaus von Apolipoprotein M oder eines mRNA-Niveaus eines Gens, welches für Apolipoprotein M aus einer biologischen Probe codiert;
(b) Vergleichen des Proteinexpressionsniveaus von Apolipoprotein M oder des mRNA-Expressionsniveaus des Gens, welches für Apolipoprotein M der biologischen Probe codiert, mit dem Proteinexpressionsniveau von Apolipoprotein M oder dem mRNA-Expressionsniveau des Gens, welches für Apolipoprotein M einer normalen Probe codiert; und
(c) Vorhersagen oder Diagnostizieren, dass Morbus Alzheimer auftritt, wenn das Proteinexpressionsniveau von Apolipoprotein M oder das mRNA-Expressionsniveau des Gens, welches für Apolipoprotein M der biologischen Probe codiert, verringerter ist, als das Proteinexpressionsniveau von Apolipoprotein M oder das mRNA-Expressionsniveau des Gens, welches für Apolipoprotein M der normalen Probe codiert.

7. Verfahren nach Anspruch 6, wobei die biologische Probe in Schritt (a) mindestens eines, ausgewählt aus der Gruppe, bestehend aus Vollblut, Serum, Plasma, Speichel, Urin, Sputum, Lymphe und Zelle, welche einer Person entnommen werden, ist.

8. Verfahren nach Anspruch 6, wobei das Verfahren zum Messen des Proteinexpressionsniveaus in Schritt (a) mindestens eines, ausgewählt aus der Gruppe, bestehend aus Western Blot, Enzyme-linked Immunosorbent Assay (ELISA), Radioimmunassay (RIA), Radioimmundiffusion, Ouchterlony-Immundiffusion, Rocket-Immunelektrophorese, immunhistochemische Färbung, Immunpräzipitation, Komplementbindungsreaktion, Fluorescenceactivated Cell Sorting (FACS) und Protein-Chip, ist.

9. Verfahren nach Anspruch 6, wobei das Verfahren zum Messen des mRNA-Expressionsniveaus in Schritt (a) mindestens eines, ausgewählt aus der Gruppe, bestehend aus Reverser Transkriptions-Polymerase-Kettenreaktion (RT-PCR), kompetitiver RT-PCR, Echtzeit-RT-PCR, Rnase Protection Assay (RPA), Northern Blot und DNA-Chip, ist.

## Revendications

1. Utilisation *in vitro* de l'apolipoprotéine M en tant que biomarqueur pour le diagnostic de la maladie d'Alzheimer.

2. Utilisation selon la revendication 1, dans laquelle un taux d'expression de l'apolipoprotéine M dans un échantillon avec la maladie d'Alzheimer est plus réduit qu'un taux d'expression de l'apolipoprotéine M dans un échantillon normal.

3. Utilisation *in vitro* d'une composition comprenant une formulation pour la mesure du taux d'expression protéinique de l'apolipoprotéine M ou d'un taux d'expression d'ARNm d'un gène codant pour l'apolipoprotéine M, pour le diagnostic de la maladie d'Alzheimer, dans laquelle, la formulation pour la mesure du taux d'expression protéinique est un anticorps spécifique de l'apolipoprotéine M, ou la formulation pour la mesure du taux d'expression d'ARNm est au moins l'un(e) choisi(e) dans le groupe constitué d'un oligonucléotide antisens, d'une amorce, et d'une sonde qui se lient spécifiquement à un gène de l'apolipoprotéine M.

4. Utilisation d'un kit comprenant la composition selon la revendication 3 pour le diagnostic de la maladie d'Alzheimer.

5. Utilisation selon la revendication 4, dans laquelle le kit est au moins l'un choisi dans le groupe constitué d'un kit de transcription inverse-réaction en chaîne de la polymérase (RT-PCR), d'un kit de puce de microréseaux, d'un kit d'ADN, et d'un kit de puce de protéines.

6. Procédé de fourniture d'informations pour le diagnostic de la maladie d'Alzheimer, le procédé comprenant :
(a) la mesure d'un taux d'expression protéinique de l'apolipoprotéine M ou d'un taux d'ARNm d'un gène codant pour l'apolipoprotéine M à partir d'un échantillon biologique ;
(b) la comparaison du taux d'expression protéinique de l'apolipoprotéine M ou du taux d'expression d'ARNm du gène codant pour l'apolipoprotéine M de l'échantillon biologique au taux d'expression protéinique de l'apolipoprotéine M ou au taux d'expression d'ARNm du gène codant pour l'apolipoprotéine M d'un échantillon normal ; et
(c) la prédiction ou le diagnostic que la maladie d'Alzheimer survient, lorsque le taux d'expression protéinique de l'apolipoprotéine M ou le taux d'expression d'ARNm du gène codant pour l'apolipoprotéine M de l'échantillon biologique est plus réduit que le taux d'expression protéinique de l'apolipoprotéine M ou le taux d'expression d'ARNm du gène codant pour l'apolipoprotéine M de l'échantillon normal.

7. Procédé selon la revendication 6, dans lequel l'échantillon biologique dans l'étape (a) est au moins l'un(e) choisi(e) dans le groupe constitué du sang total, du sérum, du plasma, de la salive, de l'urine, d'un crachat, de la lymphe, et d'une cellule, qui sont séparés d'un individu.

8. Procédé selon la revendication 6, dans lequel le procédé pour la mesure du taux d'expression protéinique dans l'étape (a) est au moins l'un(e) choisi(e) dans le groupe constitué d'un Western blot, d'un test ELISA (enzyme linked immunosorbent assay), d'un test radioimmunologique (RIA), d'une radioimmunodiffusion, d'une immunodiffusion d'Ouchterlony, d'une immunoélectrophorèse en roquettes, d'une immuno-histocoloration, d'un test d'immuno-précipitation, d'un test de fixation du complément, d'un trieur de cellules activées par fluorescence (FACS), et d'une puce de protéines.

9. Procédé selon la revendication 6, dans lequel le procédé pour la mesure du taux d'expression d'ARNm dans l'étape (a) est au moins l'un (e) choisi (e) dans le groupe constitué d'une transcription inverse-réaction en chaîne de la polymérase (RT-PCR), d'une RT-PCR compétitive, d'une RT-PCR en temps réel, d'un test de protection à la RNase (RPA), d'une analyse Northern blot, et d'une puce d'ADN.
